# EUROPEAN PATENT APPLICATION

(11) **EP 1 600 158 A1**
(43) Date of publication of application: **30.11.2005**
(21) Application number: 04291328.5
(22) Date of filing: 26.05.2004
(51) Int. Cl.: A61K 31/4178, A61P 17/10

(54) **Use of ondansetron for the treatment of inflammation, and pharmaceutical compositions thereof**

(71) Applicant: Galderma Research & Development, S.N.C., 06560 Valbonne, Sophia Antipolis (FR)
(72) Inventor: Fabrizio, Dolfi, 06560 Valbonne (FR); Tremel, Nadège, 06270 Villeneuve Loubet (FR)
(74) Representative: Bernstein, Claire Jacqueline

(57) **Abstract**

The present invention generally relates to the use of ondansetron as an active principle in the manufacture of pharmaceutical compositions, more particularly dermatological compositions, intended for a curative and/or prophylactic anti-inflammatory treatment, by the topical route. More specifically, the present invention provides the use of Ondansetron to topically treat rosacea, an inflammatory skin disorder.

## Description

The present invention generally relates to the use of ondansetron as an active principle in the manufacture of pharmaceutical compositions, more particularly dermatological compositions, intended for a curative and/or prophylactic anti-inflammatory treatment, by the topical route. More specifically, the present invention provides the use of Ondansetron to topically treat rosacea, an inflammatory skin disorder.

Ondansetron, or (±) 1, 2, 3, 9-tetrahydro-9-methyl-3-(2-methyl-1H-imidazol-1-yl)methyl]-4H-carbazol-4-one, is a product which is already known per se, and is disclosed and claimed in U.S. Patent 4,695,578. Ondansetron acts as a potent and selective antagonist of the neuronal 5HT receptor type 3, located on primary afferent nerves. Specifically, Ondansetron has been approved in the past in palliative therapy to prevent or treat chemotherapy induced nausea and vomiting. In addition, this compound is useful as an analgesic, for example, in the alleviation of pain associated with migraine, headache, and many other forms of pain for which 5HT is the endogenous mediator.

There is, however, no published data regarding the use of ondansetron, by the topical route, to treat the inflammatory skin condition, rosacea.

Thus, the subject of the present invention is the use of an anti-inflammatory effective amount of ondansetron for the manufacture of a topical pharmaceutical composition for the treatment of inflammation. Additionally, the present invention provides a novel method of treating inflammation by topically applying a pharmaceutical composition comprising ondansetron. More specifically, the present invention provides for the use of ondansetron to topically treat rosacea, an inflammatory skin disorder.

Rosacea is a common, but often overlooked, skin condition of uncertain etiology that can lead to significant facial disfigurement, ocular complications, and severe emotional distress. The progression of rosacea is variable; however, typical stages include: (1) facial flushing, in which mostly the cheeks appear to have a flush or sunburn, the redness being caused by dilation of the blood vessels in the skin, which allows more blood to flow and pool under the surface of the skin, (2) erythema and/or edema, telangiectasia, (3) pimples appearing as small red and solid (papules) or pus-filled (pustules), and (4) rhinophyma.

Central facial erythema and telangiectasis are predominant in the early stages of rosacea. This progresses to a chronic inflammatory infiltrate with central facial papules and, less commonly, pustules. Intermittent or chronic facial edema may also occur. Some patients develop rhinophyma, a coarse hypertrophy of the connective tissue and sebaceous glands of the nose evident as small knobby bumps on the nose.

The predominant, presenting complaints of rosacea are intermittent, central facial flushing and erythema. Itching is typically absent; however, many patients complain of a stinging pain (which can be severe) associated with flushing episodes. These flushing episodes are often socially embarrassing and can occur unpredictably or can be linked to environmental, chemical, food, or emotional triggers. Common triggers include exposure to the sun, cold weather, sudden emotion (laughter or embarrassment), hot beverages, and alcohol consumption.

According to the present invention, topical ondansetron (in cream, gel, or lotion form, for example) administered once or twice daily is effective for facial flushing. The present invention also teaches that topical ondansetron can reduce pustular and papular rosacea, and erythema and telangiectasis in some patients.

As a consequence, the invention relates to the use of an effective amount of ondansetron for the manufacture of a topical pharmaceutical composition for the treating rosacea, it also relates to the use of ondansetron for the treatment of rosacea where the rosacea is treated at a stage in which facial flushing is evident, and preferably, where the rosacea is treated at a stage in which a symptom selected from the group consisting of erythema, edema, telangiectasia and ocular is evident.

Rosacea is a chronic, relapsing disorder, and long-term treatment is generally required. Control of symptoms can be successfully maintained by long-term topical use of ondansetron.

Other characteristics, aspects, objectives and advantages of the invention will become still more clearly apparent on reading the description which follows and various concrete, but in no way limiting, examples intended to illustrate it.

Preferably, the pharmaceutical composition of the invention is intended for topical use. More particularly, the pharmaceutical composition is a dermatological composition.

Such a composition according to the present invention is a composition for the topical treatment of inflammation comprising an anti-inflammatory effective amount of ondansetron and a topical pharmaceutically acceptable carrier therefor.

The dermatological composition is more generally intended for the treatment, by the topical route, of skin diseases or complaints having at least a vascular lesion, one inflammatory component or, at the same time, one inflammatory and one infectious component.

More particularly, the skin diseases or complaints correspond to skin inflammations accompanying any type of dermatosis such as eczema, psoriasis, rosacea, acne vulgaris, ulcers, seborrhoeic dermatitis and irritations induced by chemical, physical or mechanical agents or others. The present invention is especially useful in the topical treatment of rosacea using ondansetron.

In what follows, "topical route" is understood to mean any technique for administration of a product by direct application to a surface (or external) part of the body, such as the skin or eye.

By the topical route, the pharmaceutical compositions based on ondansetron, which are therefore more particularly intended for the treatment of the skin or mucous membranes, can be provided in the form of ointments, creams, milks, salves, powders, impregnated pads, solutions, gels, sprays, lotions or suspensions. They can also be provided in the form of lipid or polymer vesicles or nanospheres or microspheres or in the form of polymer patches or hydrogels making possible a controlled release formulation. These compositions by the topical route can moreover be provided either in anhydrous form or in an aqueous form, according to the clinical indication. Formulations for topical use which are particularly highly suitable in the context of the implementation of the present invention are given in particular in the examples of the present specification.

In a preferred embodiment of the invention, the topical composition is in the form of a gel, a cream or a lotion.

By the ocular route, compositions of the present invention are mainly eyewashes.

The compositions, preferably for topical use, according to the invention contain ondansetron at a concentration preferably of between 0.01% and 5% by weight, more preferably 0,5, and most preferably 0,75% with respect to the total weight of the composition.

According to a specific and preferred embodiment of the present invention, the overall content of the ondansetron does not exceed 5 to 10% of the total weight of the composition. Preferably, the medicinal compositions are topically applied twice a day, for a period of 3 months.

The medicinal compositions according to the invention can, of course, additionally contain inert or even pharmacodynamically or cosmetically active additives or combinations of these additives and in particular: wetting agents; depigmenting agents such as hydroquinone, azelaic acid, caffeic acid or kojic acid; emollients; hydrating agents such as glycerol, PEG 400, thiamorpholinone and its derivatives or alternatively urea; antiseborrhoeic or anti-acne agents, such as S-carboxymethylcysteine, S-benzylcysteamine, their salts or their derivatives, or benzoyl peroxide; fungicides such as ketoconazole or 4,5-polymethylene-3-isothiazolidones; carotenoids and in particular .beta.-carotene; antipsoriatic agents such as anthralin and its derivatives; and finally 5,8,11,14-eicosatetraynoic acid and 5,8,11-eicosatriynoic acid and their esters and amides; metronidazole, ivermectine, and other agents used for trating rosacea; anti-inflammatory agents such as NSAIDS.
The compositions according to the invention can also contain flavor-improving agents, preserving agents, such as the esters of para-hydroxybenzoic acid, stabilizing agents, moisture-regulating agents, pH-regulating agents, agents for modifying osmotic pressure, emulsifying agents, UV-A and UV-B screening agents, and antioxidizing agents, such as alpha-tocopherol, butylated hydroxyanisole or butylated hydroxytoluene.

Of course, a person skilled in the art will take care to choose the optional compound(s) to be added to the pharmaceutical composition so that the advantageous properties intrinsically attached to the composition are not, or not substantially, detrimentally affected by the envisaged addition.

A number of examples intended to illustrate various concrete formulations in accordance with the invention will now be given, without implied limitation.

### EXAMPLE 1

An illustration is given here of a concrete formulation example in accordance with the invention which is provided in the form of a gel for topical use.

| | |
|---|---|
| Ondansetron | 0.75 g |
| Carbopol 980 (Goodrich) | 0.6 g |
| Polyethylene glycol 400 | 3 g |
| Sodium hydroxide | q.s. pH 5 |
| Preserving agents | q.s. |
| Demineralized water | q.s. for 100 g |

### EXAMPLE 2

An illustration is given here of a concrete formulation example in accordance with the invention which is provided in the form of a cream for topical use.

| | |
|---|---|
| Ondansetron | 0.75 g |
| Methyl glucose sesquistearate | 1 g |
| Stearyl alcohol | 0.5 g |
| Liquid paraffin oil | 6 g |
| Polyethylene glycol 400 | 2 g |
| Methyl glucose sesquistearate | 5 g |
| polyoxyethylenated with 20 mol of EO Carbopol 981 (Goodrich) | 0.4 g |
| Glycerol | 7 g |
| Cyclomethicone | 4 g |
| Sodium hydroxide | q.s. pH 5 |
| Preserving agents | q.s. |
| Demineralized water | q.s. for 100 g |

## Claims

1. Use of an anti-inflammatory effective amount of ondansetron for the manufacture of a topical pharmaceutical composition for the treatment of inflammation.

2. The use according to claim 1, wherein the amount of ondansetron present in said pharmaceutical composition ranges from about 0.01% to 5% by weight with respect to the total weight of the pharmaceutical composition.

3. The use according to claim 1, where said treatment of inflammation comprises treatment of a skin disease.

4. The use according to claim 3, wherein said skin disease is accompanied by dermatosis.

5. The use according to claim 4, wherein said dermatosis is selected from the group consisting of eczema, psoriasis, acne rosacea, acne vulgaris, ulcers, seborrhoeic dermatitides and irritations induced by chemical, physical or mechanical agents.

6. The use according to claim 4, wherein said dermatosis is rosacea.

7. Use of an effective amount of ondansetron for the manufacture of a topical pharmaceutical composition for the treatment of rosacea.

8. The use according to claim 7 where the rosacea is treated at a stage in which facial flushing is evident.

9. The use according to claim 7 where the rosacea is treated at a stage in which a symptom selected from the group consisting of erythema, edema, telangiectasia and ocular is evident.

10. A composition for the topical treatment of inflammation comprising an anti-inflammatory effective amount of ondansetron and a topical pharmaceutically acceptable carrier therefor.

11. The composition according to claim 10 in the form of a gel.

12. The composition according to claim 10 in the form of a cream.

13. The composition according to claim 10 in the form of a lotion.
